# FASCICULE DE BREVET EUROPEEN

(11) **EP 2 381 845 B1**
(45) Date de publication et mention de la délivrance du brevet: **11.09.2019**
(21) Numéro de dépôt: 09801986.2
(22) Date de dépôt: 29.12.2009
(51) Int. Cl.: A61B 5/103, A61B 5/11, G06F 3/01, G06F 3/033

(54) **DISPOSITIF, METHODE ET SYSTEME DE CARACTERISATION DE MOUVEMENTS D'UN PIED**
VORRICHTUNG, VERFAHREN UND SYSTEME ZUR CHARAKTERISIERUNG VON FUSSBEWEGUNGEN
DEVICE, METHOD AND SYSTEM FOR CHARACTERISING FOOT MOVEMENTS

(30) Priorité: 05.01.2009 US 142408 P
(43) Date de publication de la demande: 02.11.2011
(73) Titulaire: Movea S.A, 38000 Grenoble (FR); Commissariat à l'Énergie Atomique et aux Énergies Alternatives, 75015 Paris (FR)
(72) Inventeur: CARITU, Yanis, F-38134 Saint-joseph-la-riviere (FR); DE FORAS, Etienne, F-38240 Meylan (FR); GODIN, Christelle, F-38190 Brignoud (FR); AUJAY, Grégoire, F-38100 Grenoble (FR)
(74) Mandataire: Loubet, Bruno Thomas
(86) Numéro de dépôt international: PCT/EP2009/067973
(87) Numéro de publication internationale: WO 2010/076313

(56) Documents cités:
- WO-A2-2005/034751
- WO-A2-2008/061023
- US-A1- 2007 260 418
- US-A1- 2008 105 065
- US-A1- 2008 214 360

## Description

La présente invention s'applique au domaine de la capture de mouvement. Plus précisément, elle traite de la détection de pas effectués par un humain pratiquant la danse, une marche ou une course à des fins ludiques ou d'entraînement. Un exemple d'application de l'invention est le jeu "Dance Dance Révolution" (Danse, Danse, Révolution ou DDR) qui se pratique en arcade de jeux ou avec une console ou un PC. Dans le mode de réalisation actuel de ce jeu, le joueur doit exécuter une séquence de pas de danse qui lui est indiquée à l'écran, ladite séquence étant rythmée par une musique. Le joueur est debout sur un tapis souple ou rigide comportant des cases qui peuvent être alignées, disposés en rectangle ou en carré, le nombre de cases pouvant varier de 4 à 6 voire 9. Chaque case est instrumentée d'un dispositif de détection de la présence du joueur. Les consignes relatives à la séquence de pas de danse peuvent être données par des flèches indiquant la case vers laquelle le joueur doit se déplacer. Ainsi, le système peut comparer la séquence exécutée avec la séquence idéale et attribuer une note au joueur. Mais ce mode de réalisation présente l'inconvénient de nécessiter ce tapis de détection qui est encombrant et coûteux s'il est rigide et fragile et peu précis s'il est souple. De plus le tapis limite l'extension des mouvements possibles.
Pour remédier à ces inconvénients, l'invention supprime les détecteurs de présence dudit tapis et les remplace par un dispositif de capture de mouvement porté sur au moins un pied du joueur et doté des capacités de traitement permettant la détection de la position du joueur par rapport aux cases d'un tapis virtuel (dont la taille et/ou le nombre de cases peuvent être aussi grands que l'on veut), ou réel mais non instrumenté.

Des dispositifs de capture de mouvement comprenant des accéléromètres et/ou des gyromètres et/ou des magnétomètres portés par un piéton existent dans l'art antérieur, notamment pour mesurer les foulées dudit piéton. C'est le cas par exemple des dispositifs divulgués par la demande de brevet américain n°US2008/105065, les demandes de brevet internationales WO2005/034751 et WO2008/061023. Ces dispositifs ne permettent cependant pas la mesure précise, simple et robuste de la direction et du sens de déplacement d'un membre. Ils ne peuvent donc être utilisés en substitution au tapis de détection. L'invention résout ce problème en procurant un dispositif de capture de mouvement qui permet la mesure précise de la direction et du sens du déplacement d'un objet instrumenté tel que par exemple un membre d'un être humain.

A cet effet, l'invention prévoit un dispositif de caractérisation des mouvements d'un pied d'un être humain selon la revendication 1.

Avantageusement, ledit module de calcul est en outre apte à exécuter une fonction d'évaluation de la conformité des mouvements du pied par rapport à une séquence de mouvements préenregistrés.

Avantageusement, ledit module de calcul est en outre apte à exécuter une fonction de commande des déplacements d'un objet virtuel représentant le pied en mouvement réel sur un afficheur relié audit module de calcul, lesdits déplacements étant en correspondance logique et quantitative avec lesdits mouvements.

Avantageusement, le dispositif de l'invention comprend en outre au moins un magnétomètre solidaire du pied et en ce que ledit module de calcul est apte à exécuter en outre à partir des sorties dudit au moins un accéléromètre et dudit au moins un magnétomètre une fonction de détermination de l'orientation dudit pied dans au moins un plan.

Pour mettre en oeuvre le procédé, l'invention prévoit également une méthode de caractérisation des mouvements d'un pied d'un être humain selon la revendication 8.

Avantageusement, au cours de ladite étape de calcul s'exécute en outre au moins une fonction de calcul de la longueur du mouvement élémentaire dans la direction de mouvement.

Avantageusement, ladite étape de calcul comporte en outre, lorsque l'élévation de l'accéléromètre sur la chaussure est sensiblement supérieure à 20°, une étape de calibration des mesures en sortie de l'étape de capture des signaux de sortie de l'accéléromètre.

Avantageusement, ladite fonction de segmentation des pas comprend une étape de centrage sur la valeur moyenne de mesures en sortie d'au moins axe de l'accéléromètre.

Avantageusement, ladite fonction de segmentation des pas comprend en outre une étape de filtrage par calcul d'au moins une moyenne glissante desdites mesures centrées

Avantageusement, ladite fonction de segmentation des pas comprend une étape de calcul d'une norme d'au moins une valeur mesurée ou calculée en sortie de l'accéléromètre puis de comparaison de ladite norme à un seuil prédéterminé pour en déduire le début ou la fin du pas.

Avantageusement, ladite fonction de segmentation des pas ne traite que les mesures sur un horizon temporel supérieur à un valeur prédéterminée.

Avantageusement, ladite fonction de segmentation des pas ne traite que les mesures commençant à la fin d'un premier intervalle de temps choisi suivant le début d'un pas et finissant au début d'un second intervalle de temps choisi précédant la fin dudit pas.

Avantageusement, ladite fonction de détermination de la direction du pas comprend une étape de calcul des maxima des valeurs absolues des intégrales des mesures selon chacun des deux axes sensiblement parallèles au plan des pas sur l'échantillon des signaux déterminé en sortie de la fonction de segmentation des pas, puis une étape de comparaison du maximum selon un des axes avec le maximum sur l'autre axe, la direction du pas étant déterminée comme étant celle de l'axe du maximum. Avantageusement, ladite fonction de détermination de la direction du pas comprend une étape de calcul des maxima des valeurs absolues des intégrales des mesures selon chacun des deux axes sensiblement parallèles au plan des pas sur l'échantillon des signaux déterminé en sortie de la fonction de segmentation des pas, puis une étape de calcul du rapport des maxima selon les deux axes, la direction du pas étant déterminée comme formant un angle avec l'axe dont le maximum figure au dénominateur dudit rapport dont la tangente est égale audit rapport.

Avantageusement, que ladite fonction de détermination du sens du pas comprend, en sortie de l'étape de détermination de la direction du pas, une étape de détermination des signes des maxima selon lesdits deux axes, ledit signe déterminant le sens du pas dans la direction déterminée. Avantageusement, ladite fonction de calcul de la longueur du pas comprend une étape de double intégration des valeurs absolues des mesures selon la direction du pas déterminée en sortie de la fonction de détermination de la direction et du sens du pas.

L'invention divulgue également un système d'évaluation des mouvements d'un pied d'un être humain selon la revendication 19.

Le dispositif de l'invention utilise des MEMS qui sont de moins en moins chers et est donc peu coûteux à produire. Il est peu encombrant et léger. Il présente l'avantage de pouvoir être utilisé pour d'autres applications, par exemple d'autres jeux ou systèmes d'entraînement où il faut également détecter la direction de mouvement et l'orientation des pieds du joueur, tels que des jeux de simulation de promenade ou de marche quasi-statique. Le même dispositif peut être porté à la main et utilisé pour détecter les mouvements verticaux et horizontaux de ladite main utilisée pour jouer de la musique, voire pour reconnaître l'écriture du porteur. Une combinaison des dispositifs de l'invention peut également être portée sur un ou deux membres supérieurs et un ou deux membres inférieurs, ladite combinaison permettant de comparer les séquences de gestes des deux catégories de membres à des séquences pré enregistrées de gestes idéaux, notamment à des fins d'entraînement, particulièrement pour des sports ou des jeux dans lesquels la coordination des gestes desdits membres inférieurs et supérieurs est un élément fondamental de l'apprentissage. La présente invention, de par sa versatilité, procure donc des avantages importants qui ne limitent pas son application au domaine dont elle est issue du jeu DDR.

L'invention sera mieux comprise, ses différentes caractéristiques et avantages ressortiront de la description qui suit de plusieurs exemples de réalisation et de ses figures annexées dont :
- Les figures 1a et 1b représentent des exemples de positionnement de dispositifs selon l'invention dans plusieurs de ses modes de réalisation ;
- Les figures 2a et 2b représentent des exemples de capteurs et d'unité de traitement pour mettre en oeuvre l'invention dans un de ses modes de réalisation ;
- Les figures 3a à 3c représentent trois types de pas de danseur représentés en vue par le haut et détectés par le dispositif de l'invention dans un de ses modes de réalisation;
- La figure 4 représente les mêmes trois types de pas de danseur représentés en vue latérale détectés par le dispositif de l'invention dans un de ses modes de réalisation;
- La figure 5 est un organigramme des traitements effectués par le dispositif de l'invention dans un de ses modes de réalisation;
- Les figures 6a à 6f représentent 6 types de déplacement d'un marcheur correspondant à 6 positionnements différents de ses chaussures tels que détectés par le dispositif de l'invention dans un de ses modes de réalisation;
- La figure 7 représente de manière simplifiée les traitements effectués pour détecter les déplacements des figures 6a à 6f ;
- La figure 8 représente un mode de réalisation de l'invention où le dispositif est porté par une main.

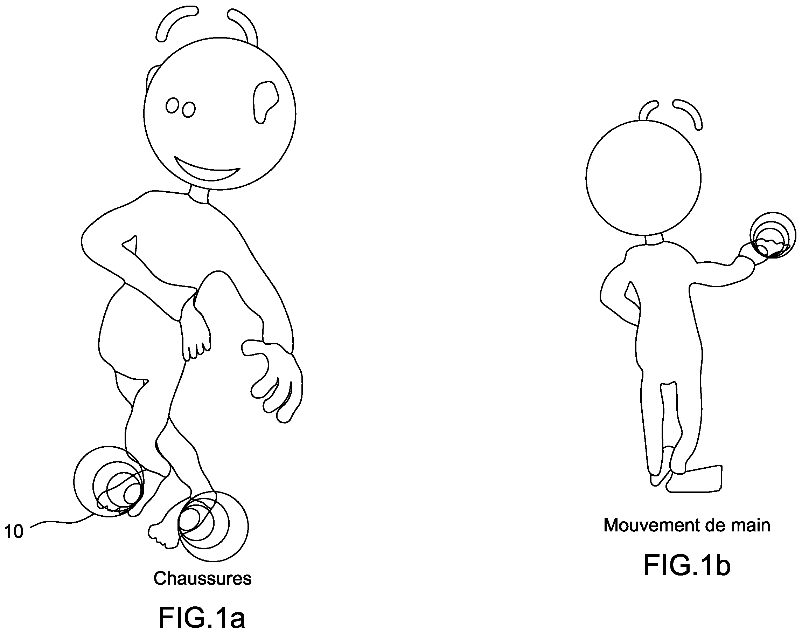

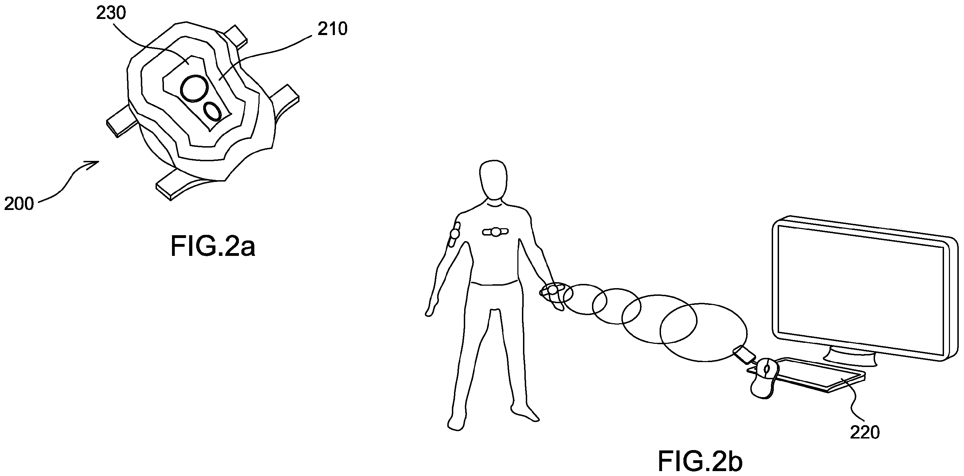

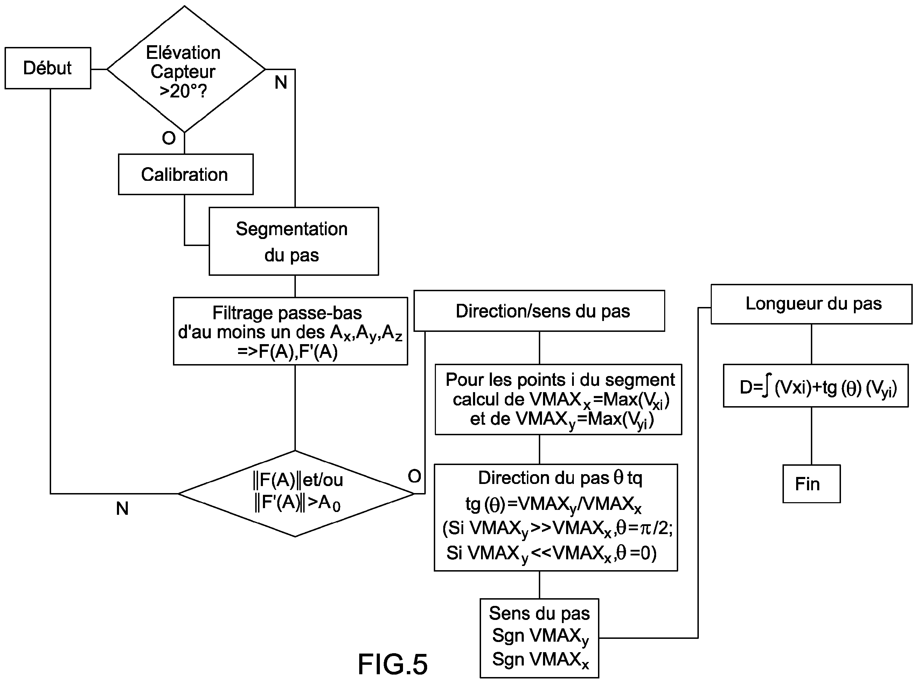

Les figures 1a et 1b représentent des exemples de positionnement de dispositifs selon l'invention dans plusieurs de ses modes de réalisation. Contrairement aux modes de réalisation d'un dispositif DDR de l'art antérieur, un joueur peut évoluer sans tapis instrumenté sous ses pieds. Sur la figure 1a, le joueur porte un capteur sur chacun de ses membres inférieurs, de préférence sous ou sur ses chaussures. Le capteur peut être incorporé dans la semelle ou fixé par des bracelets élastiques sur le dessus de ses chaussures ou, éventuellement, fixé également par des bracelets élastiques sur chacune de ses chevilles. Les types de capteurs utilisables sont indiqués en commentaire à la figure 2a. Selon le lieu de fixation des capteurs, une calibration pourra être nécessaire ou utile, comme expliqué en commentaire à la figure 5.
Des dispositifs identiques peuvent être utilisés pour jouer à d'autres jeux que le DDR, notamment des jeux de simulation de marche ou utilisant des instruments de musique, par exemple, à percussion.
Dans tous les cas, les dispositifs de capture de mouvement sont reliés à un dispositif de calcul distant du joueur et relié au dispositif du contrôle et de visualisation du jeu.
Dans d'autres scenarii de jeu, tels que celui représenté sur la figure 1b, le joueur peut porter un dispositif de capture de mouvement sur une ou deux mains dont les mouvements vont être analysés pour être comparés à un scénario guide ou pour générer une commande d'un système relié au module de calcul.

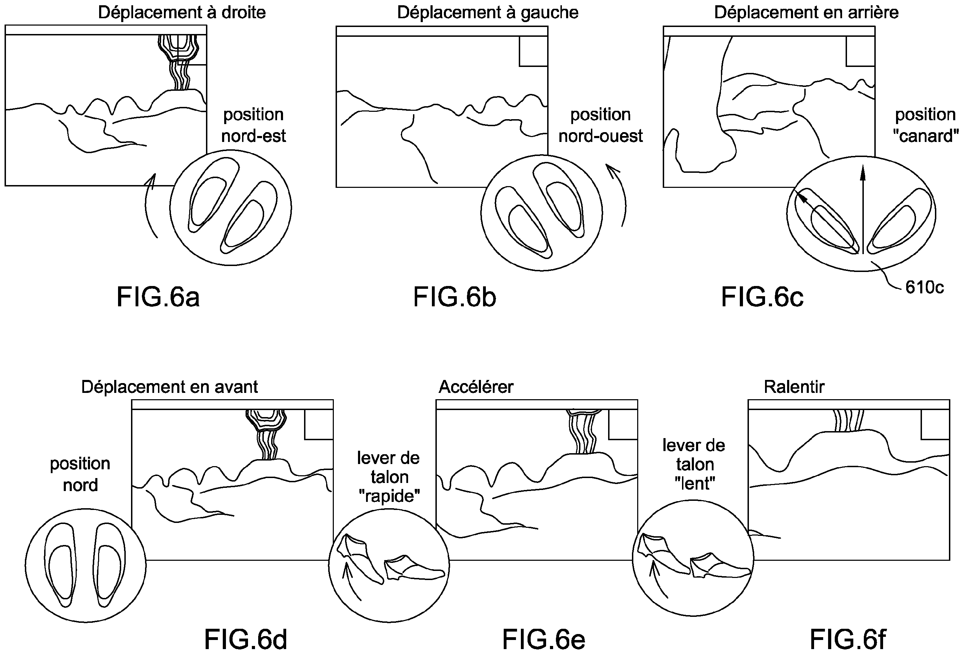

Les figures 2a et 2b représentent des exemples de capteurs et d'unité de traitement pour mettre en oeuvre l'invention dans un de des modes de réalisation.
Dans les scenarii de type DDR ou d'autre jeux, le joueur portera à des emplacements judicieusement choisis (dessous ou dessus de la chaussure, cheville, poignet, etc...) un dispositif 200 du type de celui représenté sur la figure 2a qui est un MotionPod™, ceci bien que toutes les possibilités offertes par un dispositif de ce type ne soient pas entièrement exploitées dans les modes de réalisation envisagés dans le cadre de la présente invention. Un MotionPod qui, en tant que tel, est un dispositif de l'art antérieur, comporte un accéléromètre 210 et un magnétomètre 230. Les deux capteurs sont à trois axes. Un accéléromètre mono ou bi axe peut suffire dans certains cas d'applications (DDR où toutes les cases prévues pour effectuer des pas de danse se situent sur une direction unique), un axe étant utilisé pour détecter le déplacement, un deuxième axe éventuel étant utilisé pour détecter le début d'un geste. Cependant, dans la plupart des cas, un accéléromètre à trois axes sera nécessaire pour déterminer la direction de mouvement. Un magnétomètre pourra être utilisé en combinaison avec l'accéléromètre, notamment pour déterminer l'orientation des pieds (yaw et pitch), comme on le verra en commentaire à la figure 6c. D'autres utilisations du magnétomètre sont également possibles, ce capteur présentant l'avantage par rapport à l'accéléromètre de donner accès aux mesures de lacet. Le MotionPod comporte en outre une capacité de prétraitement permettant de préformer des signaux à partir des capteurs, un module de transmission radiofréquence des dits signaux au module de traitement lui-même et une batterie. Ce capteur de mouvement est dit « 3A3M » (trois axes d'accéléromètre et trois axes de magnétomètre). Les accéléromètres et magnétomètres sont des micro capteurs du commerce de faible encombrement, de faible consommation et à bas coût, par exemple un accéléromètre trois voies de la société Kionix™ (KXPA4 3628) et des magnétomètres d'HoneyWell™ de type HMC1041Z (1 voie verticale) et HMC1042L pour les 2 voies horizontales. D'autres fournisseurs existent : Memsic™ ou Asahi Kasei™ pour les magnétomètres et STM™, Freescale™, Analog Device™ pour les accéléromètres, pour ne citer que ceux là. Dans le MotionPod, pour les 6 voies signal, il n'y a qu'un filtrage analogique et ensuite, après conversion analogique numérique (12 bits), les signaux bruts sont transmis par un protocole radiofréquence dans la bande Bluetooth™ (2,4GHz) optimisé pour la consommation dans ce type d'applications. Les données arrivent donc brutes à un contrôleur connecté au module de calcul 220 de la figure 2b. Ce contrôleur peut recevoir les données d'un ensemble de capteurs, par exemple deux capteurs, chacun étant implanté sur une des chaussures. Les données sont lues par le contrôleur et mises à disposition des logiciels. La cadence d'échantillonnage est réglable. Par défaut, elle est fixée à 200 Hz. Des valeurs plus élevées (jusqu'à 3000 Hz, voire plus) peuvent néanmoins être envisagées, permettant une plus grande précision dans la détection de chocs par exemple.
Les traitements qui permettent de mettre en oeuvre l'invention et qui seront présentés en commentaire à la figure 5 sont implantés sur le module de calcul 220 qui peut résider sur une unité centrale d'un PC du commerce, d'une console de jeu ou d'un système informatique de type arcade de jeu. L'afficheur du module de calcul permet au joueur de visualiser les consignes qui lui sont données pour exécuter le scénario du jeu, pour suivre éventuellement l'évolution de son avatar en comparaison à celle du modèle et en tout état de cause pour être informé de ses performances.

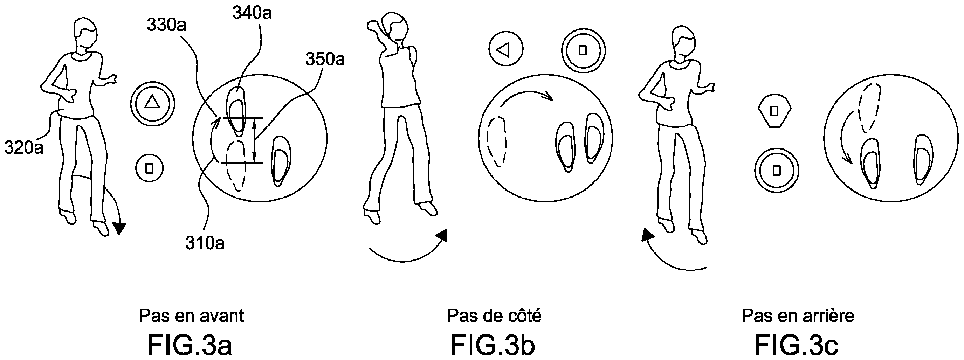

Les figures 3a à 3c représentent trois types de pas de danseur représentés en vue par le haut et détectés par le dispositif de l'invention dans un de ses modes de réalisation.
Comme on le voit sur les 3 figures 3a, 3b et 3c, les scénarii de base du jeu DDR reposent sur des pas du danseur vers l'avant (3a), de côté (3b) et vers l'arrière (3c). Sur un tapis virtuel de danse à 9 cases disposées en carré, on peut également envisager des pas en diagonale qui ne sont pas représentés ici. On peut aussi envisager d'étendre le tapis à toute la surface au sol disponible en augmentant le nombre de cases, en changeant la forme des cases ou leur taille.
Comme représenté sur la figure 3a, le pied gauche quitte la position 310a où il est d'abord posé en se levant pour rejoindre la position 320a où il se pose à nouveau. La trajectoire 310a, 320a représente la phase de vol du pied caractéristique du pas. L'arc 330a représente la direction du pas, ici vers l'avant. La flèche 340a représente le sens du pas sur la direction. La grandeur 350a représente la longueur du pas entre le lever et le poser.
Dans un dispositif DDR de l'art antérieur, la pression du joueur sur le capteur positionné sous la nouvelle position du pied 320a permet de localiser celui-ci au moment du poser du pied sur l'une des cases du tapis et de déduire ainsi le pas effectué. En revanche, dans le dispositif de jeu de danse selon l'invention, comme il est suggéré par les 3 figures 3a, 3b et 3c, le capteur porté par le pied du joueur permet de suivre la trajectoire du pied en détectant son lever et/ou son poser et en déterminant la direction, le sens et la longueur du déplacement.

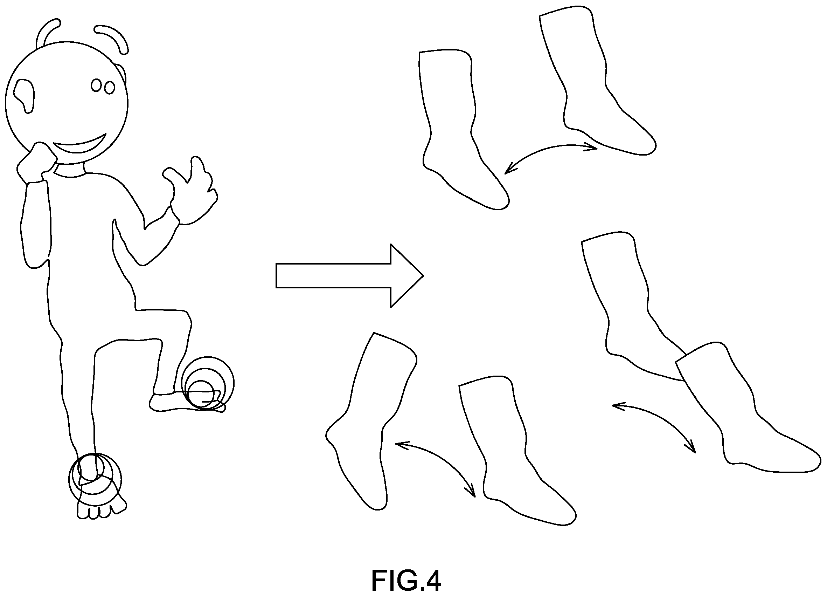

La figure 4 représente les mêmes trois types de pas de danseur représentés en vue latérale détectés par le dispositif de l'invention dans un de ses modes de réalisation.
La figure 4 décompose le lever et le poser du pied du joueur qui déterminent le début et la fin du pas. Il s'agit des deux instants qui peuvent être détectés par l'accéléromètre 210 dans les 3 cas de la figure, comme dans les autres cas. Ces deux instants sont en effet des moments de discontinuités dans les lectures de l'accéléromètre qui vont être isolés par les traitements selon l'invention qui sont explicités dans la suite de la description. Ce traitement permet la segmentation des mouvements et donc la détermination d'un pas.

La figure 5 est un organigramme des traitements effectués par le dispositif de l'invention dans un de ses modes de réalisation.
Le capteur peut être dans n'importe quelle orientation sur la chaussure, mais il peut être nécessaire d'inclure dans les traitements une étape de calibration préalable aux étapes de calcul, notamment lorsque le repère de l'accéléromètre fait un angle sensiblement supérieur à 20° avec le plan horizontal. Un exemple de calibration est décrit comme calibration anatomique ou calibration boîtier - le boîtier étant alors la chaussure - dans la demande européenne publiée sous le numéro EP1985233 et appartenant aux mêmes demandeurs. En dessous de 20°, le traitement est robuste à ce défaut d'orientation. Dans le cas contraire, l'étape de calibration permet de retrouver l'orientation du capteur sur la chaussure et de passer à des axes virtuels dans les directions liées à la chaussure. Dans les étapes suivantes, on dispose alors d'une mesure selon l'axe vertical, selon un axe horizontal orienté vers l'avant (du talon vers la pointe de la chaussure) et selon un autre axe horizontal perpendiculaire à l'axe talon pointe et orienté vers la gauche du personnage portant les chaussures.
La détermination du pas passe alors par les étapes suivantes :
- Segmentation du pas ;
- Détermination de la direction et du sens du pas ;
- Si nécessaire, détermination de la longueur du pas.

### 1/ Segmentation du pas

Comme indiqué sur la figure 4, on commence par déterminer les moments de lever et du poser du pied, ou seulement le poser. Selon le scénario applicatif, on peut utiliser les mesures d'un seul axe de l'accéléromètre, de 2 ou de 3 axes. Ces mesures sont utilisées, selon les étapes du calcul, brutes, en valeur absolue, éventuellement avec leurs dérivées.

Dans un premier mode de réalisation, seul le choc lié à l'atterrissage (poser) est utilisé pour la segmentation. Le calcul de détection de la direction sera alors lancé sur le passé, jusqu'au choc précédent.

Une au moins des données A_{X}, A_{Y} et A_{Z} qui sont reçues en sortie de l'accéléromètre 210 est traitée par le module de calcul 220.

Les étapes de poser de pied font l'objet d'une détection de choc, basée sur une norme de l'accélération : un choc est détecté si la valeur absolue ou la norme de la mesure d'accélération sur un des axes (par exemple la verticale: A_{Z}) dépasse un seuil déterminé par réglage. Entre deux chocs, le signal est enregistré, on obtient une suite de valeurs A_{X}, A_{Y} et A_{Z} appelée SA_{X}, SA_{Y} et SA_{Z}.

Dans une variante de ce mode de réalisation, pour éviter les faux rebonds, on peut ne traiter que les suites de valeurs (entre deux chocs) ayant un nombre significatif de mesures, ce qui correspond a un temps minimum pour effectuer le geste. De même, dans une variante complémentaire prévue pour améliorer la détection de la translation, une partie des signaux au début et à la fin du choc peuvent éventuellement être enlevés, car ils peuvent contenir des mesures du choc pied/sol et non pas de la translation.

Dans un second mode de réalisation, on peut également détecter par seuillage le décollage et l'atterrissage pour segmenter le pas. Dans ce mode de réalisation, on utilise une combinaison sur des accélérations selon 1, 2 ou 3 axes et les dérivées de ces valeurs et que l'on compare à un seuil déterminé par réglage. Lorsque cette combinaison dépasse le seuil pendant une durée suffisamment longue on détecte le début du pas. La fin du pas est détectée lorsqu'elle repasse en dessous du seuil pendant une durée suffisamment longue.

Dans une variante de ce mode de réalisation, on réalise un filtrage passe-bas des valeurs sur une fenêtre temporelle glissante dont la durée est également déterminée par réglage.

A la fin de cette étape, on dispose alors d'une fenêtre temporelle délimitée par un début et une fin, contenant un pas.

### 2/ Détection de la direction et du sens du pas

Dans les deux modes de réalisation de l'étape précédente, on procède de manière identique. On commence par retirer à SA_{X} et SA_{Y} leurs moyennes respectives pour trouver l'accélération propre du capteur. 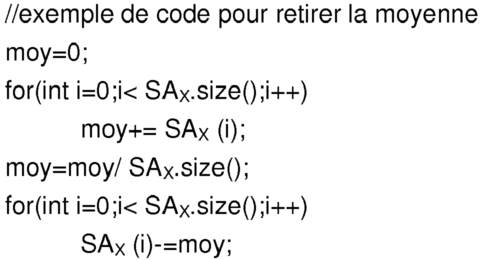 En effet, l'accélération mesurée contient l'accélération de la gravité qui s'additionne à l'accélération propre du capteur. Dans le cas d'une translation pure du capteur, sans rotation, la contribution de l'accélération de la gravité est constante. De plus, l'accélération propre a une moyenne nulle entre deux chocs, puisque la vitesse est nulle au départ et a l'arrivée. Donc :
A tout moment : A_{X}(i)=A_{XP} (i)+A_{xg}(i)
Avec A_{XP} = accélération propre et A_{XG} = accélération due à la gravité, A_{X} = accélération mesurée, integ représentant l'intégrale sur toute la fenêtre temporelle.
Alors: integ(A_{X})=integ(A_{XP})+integ(A_{XG})
Donc: integ(A_{X}=integ(A_{XG}) puisque integ(A_{XP}) =0 (étant donné que l'objet se déplace entre deux points où la vitesse est nulle, la décélération précédent le poser doit compenser strictement l'accélération suivant le lever).
S'il n'y a pas de rotation (hypothèse de pas simple), alors A_{XG} est constante et on peut écrire integ(A_{XG})=moy(A_{XG}*nbpoints)= A_{XG}(i)*nbpoints, quelque soit i. On trouve A_{XG}(i)=moy(A_{XG})
On en déduit A_{XP}(i)=A_{X}(i)-A_{XG}(i)=A_{X}(i)-moy(A_{XG})
Il ne reste plus alors qu'a intégrer A_{XP} de 0 a i pour trouver la vitesse instantanée (toujours dans l'hypothèse d'absence de rotation)
Pour ce faire, on calcule les sommes cumulées des SA_{X} et SA_{Y}, ce qui donne les vitesses instantanés VA_{X} et VA_{Y} du capteur. 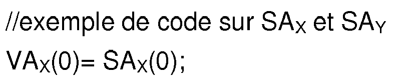 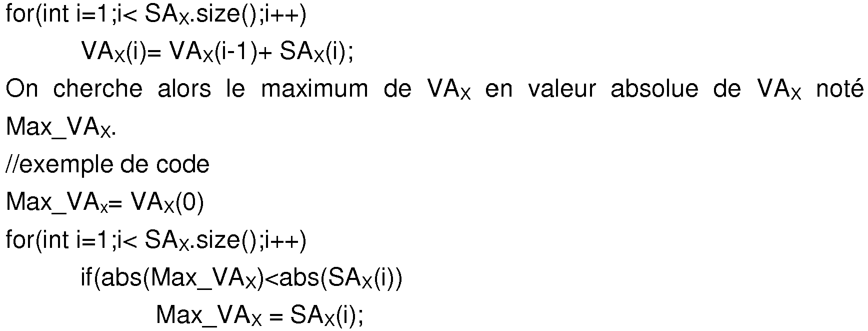 On procède ensuite de même pour VA_{Y}, le maximum de VA_{Y} en valeur absolue étant noté Max_VA_{Y}. Un exemple de code peut facilement être transposé de celui donné pour VA_{X}.
On décide alors que la direction de translation (direction du pas) est celle associée au maximum de Max_VA_{X} et de Max_VA_{Y}.
On détermine alors le sens du pas dans chaque direction par le signe de Max_VA_{X} et de Max_VA_{Y}. 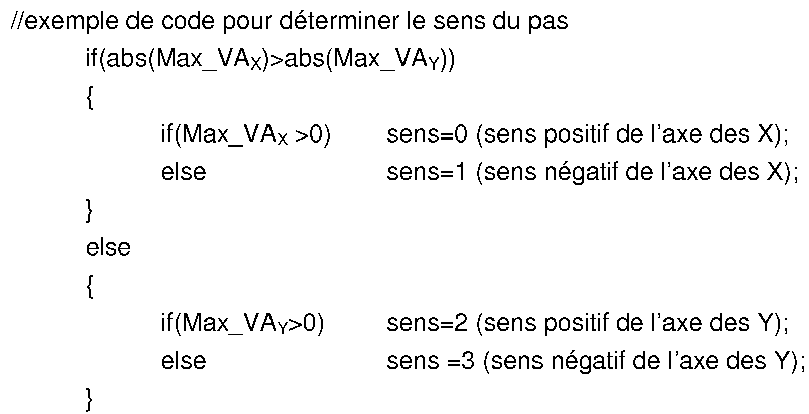 Dans une variante avantageuse de réalisation, on peut utiliser les deux suites VA_{X} et VA_{Y} ou les deux valeurs Max_VA_{X} et Max_VA_{Y} pour déterminer une direction du pas en diagonale. Dans le premier cas (traitement des suites), on peut réaliser une analyse en composantes principales pour trouver la droite de corrélation entre les deux suites qui donne la direction du pas. Dans le deuxième cas, on peut calculer le rapport des deux maxima. En première approximation, le rapport Max_VA_{Y} / Max_VA_{X} est la tangente de l'angle θ de la direction du pas avec l'axe des X. Le sens du pas peut être alors déterminé sensiblement de la même manière que ci-dessus : dans ce cas on détermine à la fois le sens sur X et sur Y (par exemple positif sur l'axe des X et négatif sur l'axe des Y) ; on donne une direction en angle mais on ne classifie pas (pas dans une case). Une fois θ calculé, on peut décider s'il s'agit d'un déplacement selon X, selon Y, ou selon XY (diagonale) : on peut diviser le cercle trigonométrique en portions entourant chaque direction : si θ<π/8 et θ>-π/8 alors c'est X ; si θ>π/8 et θ<3*π/8 alors c'est XY ; si θ>3*π/8 θ<5*π/8 alors c'est Y.
Dans une troisième variante, on peut travailler directement sur Ma_{x_}VA_{Y} et Max_VA_{X}. Si |Max_VA_{X}|>s*|Max_VA_{Y}| alors c'est X, |Max_VA_{Y}|>s*|Max_VA_{X}| alors c'est Y ; si |Max_VA_{X}|<s*|Max_VA_{Y}| avec |Max_VA_{Y}|<s*|Max_VAₓ| alors c'est XY.

Dans une autre variante de réalisation, on peut intégrer les Va_{X} et Va_{Y}, ce qui donne la position à tout instant Xaₓ et Xa_{y}, l'axe de translation choisi est alors l'axe qui a le plus grand déplacement (dans la description précédente, Va_{X} et Va_{Y} sont remplacés par les déplacements Xaₓ et Xa_{y}).
De la même manière on peut remplacer Va_{X} (et Va_{Y}) par Sa_{X} (et Sa_{Y}), la somme cumulée de |Sa_{X}| (et |Sa_{Y}|) : cumsum(|Sa_{X}|) (et cumsum(|Sa_{Y}|)). Dans ces trois cas, on remplace Max_Vax par Max_Xax ou Max_Sax ou Max(cumsum(|Sa_{X}|)), ainsi que Max_Va_{Y} par Ma_{X}_Xa_{Y} ou Ma_{X}_Sa_{Y} ou Max(cumsum(|Sa_{Y}|)).

### 3/ Calcul optionnel de la longueur du pas

Une fois la direction déterminée, on réalise une double intégration dans cette direction pour calculer une distance et sélectionner la case virtuelle dans laquelle arrive le pied, dans le cas d'un jeu DDR utilisant le dispositif et la méthode de l'invention.

Les traitements de la figure 5 ont été décrits dans l'exemple de réalisation du jeu DDR mais peuvent être utilisés dans tout contexte où il est nécessaire de déterminer une direction, un sens et une longueur de déplacement d'un capteur muni d'au moins un accéléromètre. Les traitements seront particulièrement avantageux dans tous les cas où les mouvements de l'objet portant le capteur peuvent être décomposés en segments séparés par des moments auxquels lesdits mouvements passent par un instant de pause relativement court pendant lequel la vitesse de l'objet est sensiblement nulle, ou par un choc mesurable. En effet, dans la détection des pas, on peut soit segmenter entre les pas en détectant les chocs soit segmenter en distinguant les périodes d'immobilité et de mouvement un pas étant alors un mouvement entre 2 périodes d'immobilité.

Les figures 6a à 6f représentent 6 types de déplacement d'un marcheur correspondant à 6 positionnements différents de ses chaussures tels que détectés par le dispositif de l'invention dans un de ses modes de réalisation. Sur les figures, les positions des pieds d'un marcheur qui porte sur ses chaussures deux dispositifs selon l'invention, tel que deux MotionPod comprenant chacun un accéléromètre et un magnétomètre, sont utilisées pour commander sa progression sur un parcours dit de «Hiking » dans une scène de montagne. Le scénario du jeu est agencé pour donner l'impression au joueur qu'il progresse dans une scène qu'il voit par l'intermédiaire d'une caméra virtuelle, les mouvements de progression dans la scène étant représentés par l'intermédiaire la dite caméra virtuelle et commandés par ses pieds instrumentés par les capteurs comme représenté sur les 6 figures :
- Sur la figure 6a, un mouvement des deux pieds vers la droite (Nord Est) commande un changement de direction du marcheur vers la droite ;
- Sur la figure 6b, un mouvement des deux pieds vers la gauche (Nord Ouest) commande un changement de direction du marcheur vers la gauche;
- Sur la figure 6c, un écartement des deux pieds en canard commande un déplacement du marcheur vers l'arrière (Sud);
- Sur la figure 6d, le marcheur conserve ses deux pieds parallèles et dans la direction initiale et progresse vers l'avant (Nord);
- Sur la figure 6e, le marcheur lève un de ses talons d'un angle de ptich qui doit être supérieur à une valeur prédéterminée (choisie avantageusement comme indiqué sur la figure à 20 à 30°) et sa vitesse augmente ;
- Sur la figure 6f, le marcheur lève un de ses talons d'un angle de pitch inférieur à la valeur prédéterminée indiquée ci-dessus et sa vitesse diminue.
Bien entendu, les mouvements de lacet (ou yaw) des figures 6a, 6b, 6c ou 6d et les mouvements de pitch des figures 6e et 6f se combinent pour déterminer de manière conjointe la direction et la vitesse du mouvement de progression. Si le joueur n'effectue aucun mouvement avec ses talons, il ne progresse pas dans la scène, mais son angle de vue de ladite scène se modifie en fonction de l'orientation de ses pieds.

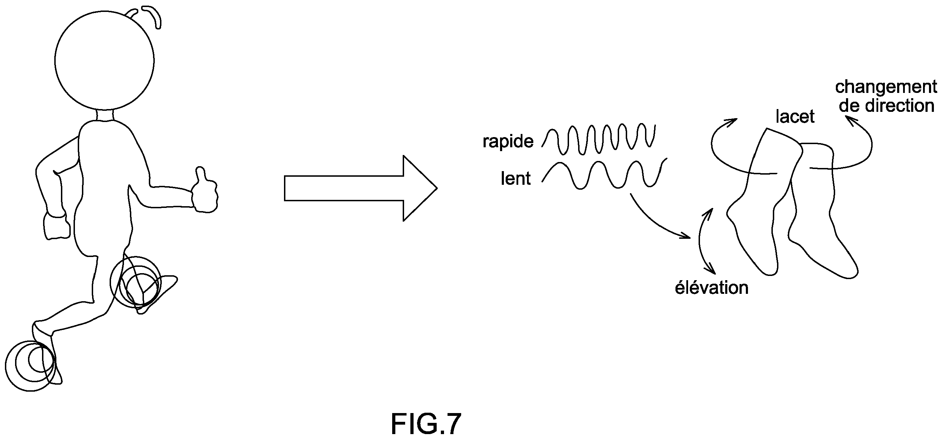

Sur la figure 6c, est représenté un angle 610c qui doit être atteint ou dépassé pour que la commande soit prise en compte. De même des seuils sont fixés pour que les commandes des autres figures soient prises en compte par les traitements qui sont explicités en commentaire à la figure 7.

La figure 7 représente de manière simplifiée les traitements effectués pour détecter les déplacements des figures 6a à 6f.
Le calcul de l'orientation des pieds est fait grâce à l'utilisation des mesures du magnétomètre en combinaison avec celles de l'accéléromètre. Cette combinaison permet un calcul d'attitude en utilisant le procédé de l'invention ayant fait l'objet de la demande de brevet PCT publiée sous le n°WO2009/127561 déposée par un des demandeurs de la présente demande. Ce procédé permet d'estimer l'orientation d'un objet dans l'espace à partir des sorties d'un accéléromètre et d'un magnétomètre par calcul d'une matrice de transfert construite à partir des mesures des deux capteurs et de leur produit vectoriel. Une autre possibilité est d'utiliser le procédé décrit dans la demande de brevet européen publiée sous le n° EP 1 985 233 également déposée par un des demandeurs de la présente demande. Ce procédé permet d'estimer un axe de rotation d'un objet en mouvement par acquisition de mesures physiques sur les trois axes d'au moins un capteur à trois instants différents.
Pour rendre le procédé plus efficace, il peut être nécessaire de réaliser une calibration du magnétomètre.

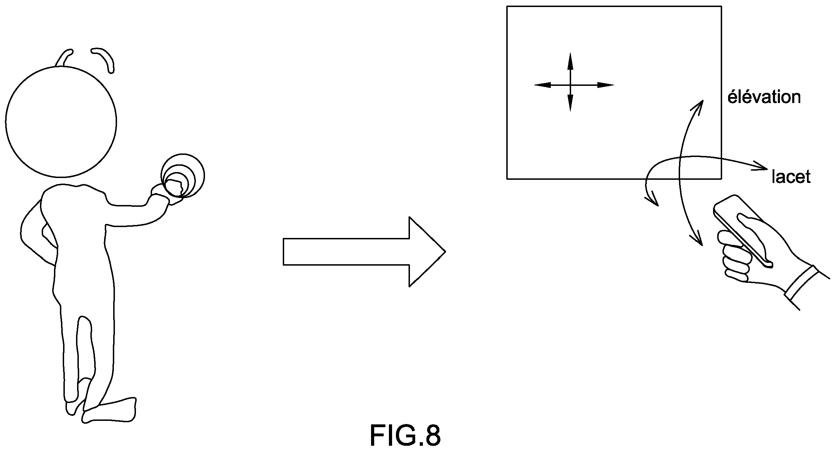

La figure 8 représente un mode de réalisation de l'invention où le dispositif est porté par une main.

Dans ce mode de réalisation, les traitements de segmentation et de détermination de la direction, du sens et de la longueur du mouvement s'appliquent aux mouvements de la main. Les traitements seront efficaces si les mouvements sont décomposables en segments séparés par des pauses relativement courtes où la main reste sensiblement immobile, comme dans le cas d'un dessin à main levée par segments ou les cas de jeu d'un instrument de musique à percussion ou de direction d'un orchestre.

Il est possible de combiner des capteurs sur les pieds et sur les jambes, notamment dans des scénarii de jeu, de simulation ou d'entraînement où la coordination des quatre membres est un élément important. De tels exemples comprennent par exemple l'entraînement au golf, au tennis ou au ski. Dans de tels cas, il pourra être nécessaire de compléter les traitements prévus par la présente invention qui permettent la caractérisation de gestes d'un type particulier par d'autres traitements qui permettent une reconnaissance de types de gestes ne présentant pas ces caractéristiques. De tels traitements de reconnaissance de geste peuvent notamment utiliser des algorithmes de type HMM (Hidden Markov Model), DTW (Dynamic Time Warping) ou LTW (Linear Time Warping). Dans les modes de réalisation divulgués notamment par les demandes de brevets français FR09/52690 et FR09/56717 déposées par les demandeurs de la présente demande. Dans les modes de réalisation décrits dans les demandes de brevets ci-dessus mentionnées, ces algorithmes présentent l'avantage de pouvoir utiliser les mêmes signaux de sortie des capteurs incorporés dans un MotionPod que les traitements de la présente invention.

Les exemples décrits ci-dessus sont donnés à titre d'illustration de modes de réalisation de l'invention. Ils ne limitent en aucune manière le champ de l'invention qui est défini par les revendications qui suivent.

## Revendications

1. Dispositif de caractérisation de mouvement d'un pied d'un être humain (10) dans un plan de marche, ledit dispositif comprenant au moins un accéléromètre (210) solidaire dudit pied et un module de calcul (220), ledit dispositif étant **caractérisé en ce que** ledit module de calcul est apte à utiliser les sorties dudit au moins un accéléromètre :
- déterminer des posés de pied ;
- segmenter lesdits mouvements en mouvements élémentaires entre une première position (310a, 310b, 310c) correspondant à un premier posé de pied et une deuxième position (320a, 320b, 320c) correspondant à un second posé de pied, et
- pour chaque mouvement élémentaire, exécuter un calcul rétroactif basé sur des données de l'accéléromètre prélevées sur un intervalle entre la première position et la deuxième position pour déterminer une direction (330a, 330b, 330c) et un sens (340a, 340b, 340c) de déplacement dudit mouvement élémentaire dans le plan de marche, ledit calcul comprenant :
- une estimation, à partir desdites données de l'accéléromètre prélevées sur l'intervalle, de données d'accélération propre de l'accéléromètre sur l'intervalle excluant l'accélération de la gravité ;
- une détermination de la direction et du sens du déplacement à partir desdites données d'accélération propre de l'accéléromètre sur l'intervalle.

2. Dispositif de caractérisation de mouvements selon la revendication 1, **caractérisé en ce que** déterminer une direction (330a, 330b, 330c) et un sens (340a, 340b, 340c) de déplacement dudit mouvement élémentaire comprend une analyse en composantes principales des projections des mesures de l'accéléromètre dans le plan de marche.

3. Dispositif de caractérisation des mouvements selon l'une des revendications 1 à 2, **caractérisé en ce que** le module de calcul (220) est apte à exécuter en outre au moins une fonction de calcul de la longueur (350a, 350b, 350c) du mouvement élémentaire dans la direction de mouvement.

4. Dispositif de caractérisation des mouvements selon la revendication 3, **caractérisé en ce que** la fonction de calcul de la longueur (350a, 350b, 350c) du mouvement élémentaire dans la direction de mouvement exécute une double intégration des projections des mesures de l'accéléromètre dans ledit plan de marche.

5. Dispositif de caractérisation des mouvements selon l'une des revendications 1 à 4, **caractérisé en ce que** ledit module de calcul est en outre apte à exécuter une fonction d'évaluation de la conformité des mouvements du pied par rapport à au moins une séquence de mouvements préenregistrés.

6. Dispositif de caractérisation des mouvements selon l'une des revendications 1 à 5, **caractérisé en ce que** ledit module de calcul est en outre apte à exécuter une fonction de commande des déplacements d'un objet virtuel représentant le pied (10) en mouvement réel sur un afficheur relié audit module de calcul (220), lesdits déplacements étant en correspondance logique et quantitative avec lesdits mouvements.

7. Dispositif de caractérisation des mouvements selon l'une des revendications 1 à 6, **caractérisé en ce qu'**il comprend en outre au moins un magnétomètre (230) solidaire du pied l'être humain et **en ce que** ledit module de calcul est apte à exécuter en outre à partir des sorties dudit au moins un accéléromètre et dudit au moins un magnétomètre une fonction de détermination de l'orientation (610c) dudit pied dans au moins un plan.

8. Méthode de caractérisation des mouvements d'un pied d'un être humain (10) dans un plan de marche, ladite méthode comprenant au moins une étape de capture des signaux de sortie d'au moins un accéléromètre (210) solidaire dudit pied et une étape de calcul par un processeur (220), ladite méthode étant **caractérisée en ce qu'**au cours de ladite étape de calcul s'exécute, à partir des sorties de l'étape de capture des signaux de sortie du au moins un accéléromètre, se déterminent des posés de pied, se segmentent lesdits mouvements en mouvements élémentaires entre une première position (310a, 310b, 310c) correspondant à un premier posé de pied et une deuxième position (320a, 320b, 320c) correspondant à un second posé de pied, et pour chaque mouvement élémentaire, s'exécute un calcul rétroactif basé sur des données de l'accéléromètre prélevées sur un intervalle entre la première position et la deuxième position pour déterminer une direction (330a, 330b, 330c) et un sens (340a, 340b, 340c) dudit mouvement élémentaire, ledit calcul comprenant :
- une estimation, à partir desdites données de l'accéléromètre prélevées sur l'intervalle, de données d'accélération propre de l'accéléromètre sur l'intervalle excluant l'accélération de la gravité ;
- une détermination de la direction et du sens du déplacement à partir desdites données d'accélération propre de l'accéléromètre sur l'intervalle.

9. Méthode de caractérisation des mouvements d'un pied selon la revendication 8, **caractérisée en ce que** au cours de ladite étape de calcul s'exécute en outre au moins une fonction de calcul de la longueur (350a, 350b, 350c) du mouvement élémentaire dans la direction de mouvement.

10. Méthode de caractérisation des mouvements d'un pied selon l'une des revendications 8 à 9, **caractérisée en ce que** ladite étape de calcul comporte en outre, lorsque l'élévation de l'accéléromètre sur la chaussure est sensiblement supérieure à 20°, une étape de calibration des mesures en sortie de l'étape de capture des signaux de sortie de l'accéléromètre.

11. Méthode de caractérisation des mouvements d'un pied selon l'une des revendications 8 à 10, **caractérisée en ce que** segmenter des mouvements élémentaires comprend une étape de centrage sur la valeur moyenne de mesures en sortie d'au moins axe de l'accéléromètre.

12. Méthode de caractérisation des mouvements d'un pied selon la revendication 11, **caractérisée en ce que** segmenter des mouvements élémentaires comprend en outre une étape de filtrage par calcul d'au moins une moyenne glissante desdites mesures centrées

13. Méthode de caractérisation des mouvements d'un pied selon l'une des revendications 8 à 12, **caractérisée en ce que** segmenter des mouvements élémentaires comprend une étape de calcul d'une norme d'au moins une valeur mesurée ou calculée en sortie de l'accéléromètre (210) puis de comparaison de ladite norme à un seuil prédéterminé pour en déduire le début ou la fin du mouvement élémentaire.

14. Méthode de caractérisation des mouvements d'un pied selon la revendication 13, **caractérisée en ce que** segmenter des mouvements élémentaires ne traite que les mesures sur un horizon temporel supérieur à une valeur prédéterminée.

15. Méthode de caractérisation des mouvements d'un pied selon la revendication 14, **caractérisée en ce que** segmenter des mouvements élémentaires ne traite que les mesures commençant à la fin d'un premier intervalle de temps choisi suivant le début d'un mouvement élémentaire et finissant au début d'un second intervalle de temps choisi précédant la fin dudit mouvement élémentaire.

16. Méthode de caractérisation des mouvements d'un pied selon l'une des revendications 13 à 15, **caractérisée en ce que** déterminer la direction du mouvement élémentaire comprend une étape de calcul des maxima des valeurs absolues des intégrales des mesures selon chacun des deux axes sensiblement parallèles au plan des mouvements élémentaires sur l'échantillon des signaux déterminé en sortie de la segmentation des mouvements élémentaires, puis une étape de comparaison du maximum selon un des axes avec le maximum sur l'autre axe, la direction du mouvement élémentaire étant déterminée comme étant celle de l'axe du maximum.

17. Méthode de caractérisation des mouvements d'un pied selon l'une des revendications 13 à 15, **caractérisée en ce que** déterminer la direction du mouvement élémentaire comprend une étape de calcul des maxima des valeurs absolues des intégrales des mesures selon chacun des deux axes sensiblement parallèles au plan des mouvements élémentaires sur l'échantillon des signaux déterminé en sortie de la segmentation des mouvements élémentaires, puis une étape de calcul du rapport des maxima selon les deux axes, la direction du mouvement élémentaire étant déterminée comme formant un angle avec l'axe dont le maximum figure au dénominateur dudit rapport dont la tangente est égale audit rapport.

18. Méthode de caractérisation des mouvements d'un pied selon l'une des revendications 16 à 17, **caractérisée en ce que** déterminer le sens du mouvement élémentaire comprend, en sortie de l'étape de détermination de la direction du mouvement élémentaire, une étape de détermination des signes des maxima selon lesdits deux axes, ledit signe déterminant le sens du mouvement élémentaire dans la direction déterminée.

19. Système d'évaluation des mouvements d'un pied d'un être humain dans un plan de marche, ledit système comprenant :
- un scénario guide de mouvements de référence à effectuer par ledit pied,
- une interface de commande desdits mouvements de référence audit pied,
- au moins un premier capteur de champ uniforme, ledit capteur solidaire dudit pied,
- un calculateur configuré pour exécuter les actions suivantes à partir des sorties du premier capteur :
• déterminer des posés de pied ;
• segmenter lesdits mouvements en mouvements élémentaires entre une première position correspondant à un premier posé de pied et une deuxième position correspondant à un second posé de pied, et
• pour chaque mouvement élémentaire, exécuter un calcul rétroactif basé sur des données de l'accéléromètre prélevées sur un intervalle entre la première position et la deuxième position pour déterminer une direction (330a, 330b, 303c) et un sens (340a, 340b, 340c) de déplacement dudit mouvement élémentaire dans le plan de marche, ledit calcul comprenant :
- une estimation, à partir desdites données de l'accéléromètre prélevées sur l'intervalle, de données d'accélération propre de l'accéléromètre sur l'intervalle excluant l'accélération de la gravité ;
- une détermination de la direction et du sens du déplacement à partir desdites données d'accélération propre de l'accéléromètre sur l'intervalle ;
lesdits mouvements dudit pied étant effectués en réponse à la commande desdits mouvements de référence.

## Patentansprüche

1. Vorrichtung zum Charakterisieren von Bewegungen eines Fußes eines Menschen (10) in einer Gehebene, wobei die Vorrichtung wenigstens einen fest mit dem Fuß verbundenen Beschleunigungsmesser (210) und ein Rechenmodul (220) umfasst, wobei die Vorrichtung **dadurch gekennzeichnet ist, dass** das Rechenmodul die Ausgänge des wenigstens einen Beschleunigungsmessers nutzen kann:
- zum Bestimmen von Fußstellungen;
- zum Segmentieren der Bewegungen in Elementarbewegungen zwischen einer ersten Position (310a, 310b, 310c) entsprechend einer ersten Fußstellung und einer zweiten Position (320a, 320b, 320c) entsprechend einer zweiten Fußstellung, und
- zum Ausführen, für jede Elementarbewegung, einer retroaktiven Berechnung auf der Basis von in einem Intervall zwischen der ersten Position und der zweiten Position gewonnenen Beschleunigungsmesserdaten zum Bestimmen einer Fortbewegungsrichtung (330a, 330b, 330c) und eines Fortbewegungssinns (340a, 340b, 340c) der Elementarbewegung in der Gehebene, wobei die Berechnung Folgendes beinhaltet:
- Schätzen, auf der Basis der in dem Intervall gewonnenen Beschleunigungsmesserdaten, von Eigenbeschleunigungsdaten des Beschleunigungsmessers in dem Intervall unter Ausschluss der Beschleunigung der Schwerkraft;
- Bestimmen der Richtung oder des Sinns der Fortbewegung auf der Basis der Eigenbeschleunigungsdaten des Beschleunigungsmessers in dem Intervall.

2. Vorrichtung zum Charakterisieren von Bewegungen nach Anspruch 1, **dadurch gekennzeichnet, dass** das Bestimmen einer Richtung (330a, 330b, 330c) und eines Sinns (340a, 340b, 340c) der Fortbewegung der Elementarbewegung das Analysieren von Projektionen von Messwerten des Beschleunigungsmessers in der Gehebene in Hauptbestandteile beinhaltet.

3. Vorrichtung zum Charakterisieren von Bewegungen nach einem der Ansprüche 1 bis 2, **dadurch gekennzeichnet, dass** das Rechenmodul (220) ferner wenigstens eine Funktion des Berechnens der Länge (350a, 350b, 350c) der Elementarbewegung in der Bewegungsrichtung ausführen kann.

4. Vorrichtung zum Charakterisieren von Bewegungen nach Anspruch 3, **dadurch gekennzeichnet, dass** die Funktion des Berechnens der Länge (350a, 350b, 350c) der Elementarbewegung in der Bewegungsrichtung eine Doppelintegration von Projektionen von Messwerten des Beschleunigungsmessers in der Gehebene ausführt.

5. Vorrichtung zum Charakterisieren von Bewegungen nach einem der Ansprüche 1 bis 4, **dadurch gekennzeichnet, dass** das Rechenmodul ferner eine Funktion des Beurteilens der Konformität der Bewegungen des Fußes mit Bezug auf wenigstens eine Sequenz von vorgespeicherten Bewegungen ausführen kann.

6. Vorrichtung zum Charakterisieren von Bewegungen nach einem der Ansprüche 1 bis 5, **dadurch gekennzeichnet, dass** das Rechenmodul ferner eine Funktion des Steuerns von Fortbewegungen eines den Fuß (10) in reeller Bewegung repräsentierenden virtuellen Objekts auf einem Display ausführen kann, verbunden mit dem Rechenmodul (220), wobei die Fortbewegungen in logischer und quantitativer Korrespondenz mit den Bewegungen sind.

7. Vorrichtung zum Charakterisieren von Bewegungen nach einem der Ansprüche 1 bis 6, **dadurch gekennzeichnet, dass** sie ferner wenigstens einen mit dem Fuß des Menschen fest verbundenen Magnetometers (230) umfasst, und dadurch, dass das Rechenmodul ferner auf der Basis der Eingänge des wenigstens einen Beschleunigungsmessers und des wenigstens einen Magnetometers eine Funktion des Bestimmens der Orientierung (610c) des Fußes in wenigstens einer Ebene ausführen kann.

8. Verfahren zum Charakterisieren von Bewegungen eines Fußes eines Menschen (10) in einer Gehebene, wobei das Verfahren wenigstens einen Schritt des Erfassens von Ausgangssignalen von wenigstens einem fest mit dem Fuß verbundenen Beschleunigungsmesser (210) und einen Schritt des Berechnens durch einen Prozessor (220) beinhaltet, wobei das Verfahren **dadurch gekennzeichnet ist, dass** im Verlaufe des ersten Rechenschritts auf der Basis der Ausgänge des Schritts zum Erfassen von Ausgangssignalen des wenigstens einen Beschleunigungsmessers der Rechenschritt ausgeführt wird, die Fußstellungen bestimmt werden, die Bewegungen in Elementarbewegungen zwischen einer ersten Position (310a, 310b, 310c) entsprechend einer ersten Fußstellung und einer zweiten Position (320a, 320b, 320c) entsprechend einer zweiten Fußstellung segmentiert werden, und für jede Elementarbewegung eine retroaktive Berechnung auf der Basis von in einem Intervall zwischen der ersten Position und der zweiten Position gewonnenen Beschleunigungsmesserdaten ausgeführt wird, um eine Richtung (330a, 330b, 330c) und einen Sinn (340a, 340b, 340c) der Elementarbewegung zu bestimmen, wobei die Berechnung Folgendes beinhaltet:
- Schätzen, auf der Basis der in dem Intervall gewonnenen Beschleunigungsmesserdaten, von Eigenbeschleunigungsdaten des Beschleunigungsmessers in dem Intervall unter Ausschluss der Beschleunigung der Schwerkraft;
- Bestimmen der Richtung und des Sinns der Fortbewegung auf der Basis der Eigenbeschleunigungsdaten des Beschleunigungsmessers in dem Intervall.

9. Verfahren zum Charakterisieren von Bewegungen eines Fußes nach Anspruch 8, **dadurch gekennzeichnet, dass** im Laufe des Schrittes des Berechnens ferner wenigstens eine Funktion des Berechnens der Länge (350a, 350b, 350c) der Elementarbewegung in der Bewegungsrichtung ausgeführt wird.

10. Verfahren zum Charakterisieren von Bewegungen eines Fußes nach einem der Ansprüche 8 bis 9, **dadurch gekennzeichnet, dass** der Rechenschritt ferner, wenn die Höhe des Beschleunigungsmessers am Schuh erheblich größer ist als 20°, einen Schritt des Kalibrierens der Messwerte am Ausgang des Schrittes des Erfassens von Ausgangssignalen des Beschleunigungsmessers beinhaltet.

11. Verfahren zum Charakterisieren von Bewegungen eines Fußes nach einem der Ansprüche 8 bis 10, **dadurch gekennzeichnet, dass** das Segmentieren der Elementarbewegungen einen Schritt des Zentrierens von Messwerten am Ausgang von wenigstens einer Beschleunigungsmesserachse auf dem Mittelwert beinhaltet.

12. Verfahren zum Charakterisieren von Bewegungen eines Fußes nach Anspruch 11, **dadurch gekennzeichnet, dass** das Segmentieren von Elementarbewegungen ferner einen Schritt des Filterns durch Berechnen von wenigstens einem gleitenden Mittelwert der zentrierten Messwerte beinhaltet.

13. Verfahren zum Charakterisieren von Bewegungen eines Fußes nach einem der Ansprüche 8 bis 12, **dadurch gekennzeichnet, dass** das Segmentieren von Elementarbewegungen einen Schritt des Berechnens einer Norm von wenigstens einem am Ausgang des Beschleunigungsmessers (210) gemessenen oder berechneten Wert und dann des Vergleichens der Norm mit einem vorbestimmten Schwellenwert beinhaltet, um davon den Anfang oder das Ende der Elementarbewegung abzuleiten.

14. Verfahren zum Charakterisieren von Bewegungen eines Fußes nach Anspruch 13, **dadurch gekennzeichnet, dass** das Segmentieren von Elementarbewegungen nur die Messwerte auf einem Zeithorizont verfolgt, der größer ist als ein vorbestimmter Wert.

15. Verfahren zum Charakterisieren von Bewegungen eines Fußes nach Anspruch 14, **dadurch gekennzeichnet, dass** das Segmentieren von Elementarbewegungen nur die Messwerte beginnend am Ende eines ersten Zeitintervalls, ausgewählt nach Beginn einer Elementbewegung, und endend am Anfang eines zweiten gewählten Zeitintervalls vor dem Ende der Elementarbewegung verfolgt.

16. Verfahren zum Charakterisieren von Bewegungen eines Fußes nach einem der Ansprüche 13 bis 15, **dadurch gekennzeichnet, dass** das Bestimmen der Richtung der Elementarbewegung einen Schritt des Berechnens der Maxima von absoluten Werten der Integrale der Messwerte gemäß jeder von zwei Achsen im Wesentlichen parallel zur Ebene der Elementarbewegungen an der bestimmten Signalprobe am Ausgang der Segmentierung der Elementarbewegungen, dann einen Schritt des Vergleichens des Maximums gemäß einer der Achsen mit dem Maximum auf der anderen Achse beinhaltet, wobei die Richtung der Elementarbewegung als die der Achse des Maximums bestimmt wird.

17. Verfahren zum Charakterisieren von Bewegungen eines Fußes nach einem der Ansprüche 13 bis 15, **dadurch gekennzeichnet, dass** das Bestimmen der Richtung der Elementarbewegung einen Schritt des Berechnens der Maxima von absoluten Werten der Integrale der Messwerte gemäß jeder von zwei Achsen im Wesentlichen parallel zur Ebene der Elementarbewegungen auf der bestimmten Signalprobe am Ausgang der Segmentierung der Elementarbewegungen, dann einen Schritt des Berechnens des Verhältnisses der Maxima gemäß den zwei Achsen beinhaltet, wobei die Richtung der Elementarbewegung als einen Winkel mit der Achse bildend bestimmt wird, deren Maximum den Nenner des Verhältnisses bildet, deren Tangente gleich dem Verhältnis ist.

18. Verfahren zum Charakterisieren von Bewegungen eines Fußes nach einem der Ansprüche 16 bis 17, **dadurch gekennzeichnet, dass** das Bestimmen des Sinns der Elementarbewegung am Ausgang des Schrittes des Bestimmens der Richtung der Elementarbewegung einen Schritt des Bestimmens der Vorzeichen der Maxima gemäß den beiden Achsen beinhaltet, wobei das Vorzeichen den Sinn der Elementarbewegung in der bestimmten Richtung bestimmt.

19. System zum Beurteilen von Bewegungen eines Fußes eines Menschen in einer Gehebene, wobei das System Folgendes umfasst:
- ein Leitszenario von durch den Fuß auszuführenden Referenzbewegungen,
- eine Schnittstelle zum Steuern der Referenzbewegungen des Fußes,
- wenigstens einen ersten einheitlichen Feldstärkesensor, wobei der Sensor fest mit dem Fuß verbunden ist,
- einen Rechner, konfiguriert zum Ausführen der folgenden Aktionen auf der Basis der Ausgänge des ersten Sensors:
• Bestimmen der Fußstellungen;
• Segmentieren der Bewegungen in Elementarbewegungen zwischen einer ersten Position entsprechend einer ersten Fußstellung und einer zweiten Position entsprechend einer zweiten Fußstellung, und
• Ausführen, für jede Elementarbewegung, einer retroaktiven Berechnung auf der Basis der in einem Intervall zwischen der ersten Position und der zweiten Position gewonnenen Beschleunigungsmesserdaten zum Bestimmen einer Richtung (330a, 330b, 330c) und eines Sinns (340a, 340b, 340c) der Fortbewegung der Elementarbewegung in der Gehebene, wobei die Berechnung Folgendes umfasst:
- Schätzen, auf der Basis der in dem Intervall gewonnenen Beschleunigungsmesserdaten, von reinen Beschleunigungsdaten des Beschleunigungsmessers in dem Intervall unter Ausschluss der Beschleunigung der Schwerkraft;
- Bestimmen der Richtung und des Sinns der Fortbewegung auf der Basis der Eigenbeschleunigungsdaten des Beschleunigungsmessers in dem Intervall;
wobei die Bewegungen des Fußes als Reaktion auf die Steuerung der Referenzbewegungen erfolgen.

## Claims

1. A device for characterizing movements of a foot of a human being (10) in a walking plane, said device comprising at least one accelerometer (210) fastened to said foot and a computation module (220), said device being **characterized in that** the computation module is capable of using the outputs of said at least one accelerometer for:
- determining foot poses;
- segmenting said movements into elementary movements between a first position (310a, 310b, 310c) corresponding to a first foot pose and a second position (320a, 320b, 320c) corresponding to a second foot pose, and
- for each elementary movement, executing at least one retroactive calculation based on accelerometer data that are taken over an interval between the first position and the second position to determine a displacement direction (330a, 330b, 330c) and line (340a, 340b, 340c) of said elementary movement in the walking plane, said calculation comprising:
- an estimation, from said accelerometer data that are taken over the interval, of acceleration data of the accelerometer on the interval excluding the acceleration of the gravity;
- a determination of the displacement direction and line from said acceleration data of the accelerometer on the interval.

2. The device for characterizing movements as claimed in claim 1, **characterized in that** determining a displacement direction (330a, 330b, 330c) and line (340a, 340b, 340c) of said elementary movement comprises a main component analysis of the projections of the accelerometer measurements in the walking plane.

3. The device for characterizing movements as claimed in one of the claims 1 to 2, **characterized in that** the computation module (220) is capable of also executing at least one function for calculating the length (350a, 350b, 350c) of the elementary movement in the direction of movement.

4. The device for characterizing movements as claimed in claim 3, **characterized in that** the function for calculating the length (350a, 350b, 350c) of the elementary movement in the direction of movement performs a double integration of the projections of the accelerometer measurements in said walking plane.

5. The device for characterizing movements as claimed in one of the claims 1 to 4, **characterized in that** said computation module is further capable of executing a function for assessing the conformity of the movements of the foot relative to at least one sequence of pre-recorded movements.

6. The device for characterizing movements as claimed in one of the claims 1 to 5, **characterized in that** said computation module is further capable of executing a function for controlling the displacements of a virtual object representing the foot (10) in real movement on a display linked to said computation module (220), said displacements logically and quantitatively corresponding to said movements.

7. The device for characterizing movements as claimed in one of the claims 1 to 6, **characterized in that** it further comprises at least one magnetometer (230) fastened to the foot of the human being and **in that** said computation module is capable of executing, also from the outputs of said at least one accelerometer and of said at least one magnetometer, a function for determining the orientation (610c) of said foot in at least one plane.

8. A method for characterizing movements of a foot of a human being (10) in a walking plane, said method comprising at least one step for capturing the output signals of at least one accelerometer (210) fastened to said foot and a step for calculation by a processor (220), said method being **characterized in that**, during said calculation step, foot poses are determined from the outputs of the step for capturing the output signals of the at least one accelerometer, said movements are segmented into elementary movements between a first position (310a, 310b, 310c) corresponding to a first foot pose and a second position (320a, 320b, 320c) corresponding to a second foot pose, and, for each elementary movement, a retroactive calculation is executed based on accelerometer data that are taken over an interval between the first position and the second position to determine a direction (330a, 330b, 330c) and a line (340a, 340b, 340c) of said elementary movement, said calculation comprising:
- an estimation, from said accelerometer data that are taken over the interval, of acceleration data of the accelerometer on the interval excluding the acceleration of the gravity;
- a determination of the displacement direction and line from said acceleration data of the accelerometer on the interval.

9. The method for characterizing movements of a foot as claimed in claim 8, **characterized in that**, during said calculation step, at least one function for calculating the length (350a, 350b, 350c) of the elementary movement in the direction of movement is also executed.

10. The method for characterizing movements of a foot as claimed in one of the claims 8 to 9, **characterized in that** said calculation step also comprises, when the elevation of the accelerometer over the shoe is substantially greater than 20°, a step for calibrating the measurements output from the step for capturing the output signals from the accelerometer.

11. The method for characterizing movements of a foot as claimed in one of the claims 8 to 10, **characterized in that** said elementary movement segmentation comprises a step for centring on the average value of measurements output from at least one axis of the accelerometer.

12. The method for characterizing movements of a foot as claimed in claim 11, **characterized in that** said elementary movement segmentation also comprises a filtering step based on calculation of at least one sliding average of said centred measurements.

13. The method for characterizing movements of a foot as claimed in one of the claims 8 to 12, **characterized in that** said elementary movement segmentation comprises a step for calculating a norm of at least one measured or calculated value output from the accelerometer (210) then for comparing said norm to a predetermined threshold to deduce the start or the end of the elementary movement therefrom.

14. The method for characterizing movements of a foot as claimed in claim 13, **characterized in that** said elementary movement segmentation processes only the measurements over a time horizon greater than a predetermined value.

15. The method for characterizing movements of a foot as claimed in claim 14, **characterized in that** said elementary movement segmentation processes only the measurements beginning at the end of a first chosen time interval following the start of an elementary movement and ending at the start of a second chosen time interval preceding the end of said elementary movement.

16. The method for characterizing movements of a foot as claimed in one of the claims 13 to 15, **characterized in that** determining the direction of the elementary movement comprises a step for calculating the maxima of the absolute values of the integrals of the measurements along each of the two axes substantially parallel to the plane of the elementary movements on the signal sample determined at the output of the elementary movement segmentation, then a step for comparing the maximum along one of the axes with the maximum on the other axis, the direction of the elementary movement being determined as being that of the axis of the maximum.

17. The method for characterizing movements of a foot as claimed in one of the claims 13 to 15, **characterized in that** determining the direction of the elementary movement comprises a step for calculating the maxima of the absolute values of the integrals of the measurements along each of the two axes substantially parallel to the plane of the elementary movements on the signal sample determined at the output of the elementary movement segmentation, then a step for calculating the ratio of the maxima along the two axes, the direction of the elementary movement being determined as forming an angle with the axis whose maximum is the denominator of said ratio for which the tangent is equal to said ratio.

18. The method for characterizing movements of a foot as claimed in one of the claims 16 to 17, **characterized in that** determining the line of the elementary movement comprises, at the output of the step for determining the direction of the elementary movement, a step for determining the signs of the maxima along said two axes, said sign determining the line of the elementary movement in the determined direction.

19. System for evaluating the movements of a foot of a human being in a walking plane, said system comprising:
- a guide scenario of reference movements to be executed by said foot,
- a control interface of said reference movements of said foot,
- at least one first uniform field sensor, said sensor being fastened to said foot,
- a calculator configured to execute the following actions from the output of said first sensor:
• determining the foot poses;
• segmenting said movements into elementary movements between a first position corresponding to a first foot pose and a second position corresponding to a second foot pose, and
• for each elementary movement, executing at least one retroactive calculation based on accelerometer data that are taken over an interval between the first position and the second position to determine a displacement direction (330a, 330b, 330c) and line (340a, 340b, 340c) of said elementary movement in the walking plane, said calculation comprising:
- an estimation, from said accelerometer data that are taken over the interval, of proper acceleration data of the accelerometer on the interval excluding the acceleration of the gravity;
- a determination of the displacement direction and line from said acceleration data of the accelerometer on the interval;
wherein said movements of the foot are executed in response to the control of said reference movements.
